# EUROPEAN PATENT SPECIFICATION

(11) **EP 3 858 346 B1**
(45) Date of publication and mention of the grant of the patent: **25.12.2024**
(21) Application number: 19865916.1
(22) Date of filing: 20.09.2019
(51) Int. Cl.: A61K 31/403, A61P 29/00, A61P 9/10, A61P 13/12, A61P 11/00, A61P 1/16

(54) **CHIGLITAZAR FOR USE IN THE TREATMENT OF FIBROSIS**
CHIGLITAZAR ZUR VERWENDUNG BEI DER BEHANDLUNG VON FIBROSE
CHIGLITAZAR POUR UTILISATION DANS LE TRAITEMENT DE LA FIBROSE

(30) Priority: 25.09.2018 CN 201811114946
(43) Date of publication of application: 04.08.2021
(73) Proprietor: Shenzhen Chipscreen Biosciences Co., Ltd., Shenzhen, Guangdong 518057 (CN)
(72) Inventor: LU, Xianping, Shenzhen, Guangdong 518057 (CN); NING, Zhiqiang, Shenzhen, Guangdong 518057 (CN); PAN, Desi, Shenzhen, Guangdong 518057 (CN); KONG, Yidi, Shenzhen, Guangdong 518057 (CN)
(74) Representative: Lavoix
(86) International application number: PCT/CN2019/106893
(87) International publication number: WO 2020/063463

(56) References cited:
- WO-A1-2011/002012
- WO-A1-2015/189401
- WO-A1-2019/024776
- CN-A- 1 562 970
- CN-A- 102 056 606
- CN-A- 102 883 721
- US-A1- 2017 174 718
- PING-PING LI ET AL: "The PPAR[alpha]/[gamma] dual agonist chiglitazar improves insulin resistance and dyslipidemia in MSG obese rats", BRITISH JOURNAL OF PHARMACOLOGY, WILEY-BLACKWELL, UK, vol. 148, no. 5, 29 January 2009 (2009-01-29), pages 610 - 618, XP071146418, ISSN: 0007-1188, DOI: 10.1038/SJ.BJP.0706745
- ZARDI E M ET AL: "Hepatic PPARs: Their Role in Liver Physiology, Fibr osis and Tr eatment", 1 January 2013 (2013-01-01), XP055918234, Retrieved from the Internet <URL:https://arts.units.it/retrieve/handle/11368/2768340/131844/013%20PPARs.pdf> [retrieved on 20220504]
- DUBOIS VANESSA ET AL: "Distinct but complementary contributions of PPAR isotypes to energy homeostasis", THE JOURNAL OF CLINICAL INVESTIGATION, vol. 127, no. 4, 3 April 2017 (2017-04-03), GB, pages 1202 - 1214, XP055918586, ISSN: 0021-9738, Retrieved from the Internet <URL:https://www.ncbi.nlm.nih.gov/pmc/articles/PMC5373878/pdf/jci-127-88894.pdf> DOI: 10.1172/JCI88894

## Description

This application claims the priority of Chinese Patent Application No. 201811114946.5, filed with the China National Intellectual Property Administration on September 25, 2018, titled "APPLICATION OF CHIGLITAZAR AND RELATED COMPOUNDS THEREOF"

### FIELD

The present disclosure relates to the field of medicine technology, and specifically relates to the application of chiglitazar and related compounds thereof.

### BACKGROUND

Chiglitazar is an active product of sodium chiglitazar. Sodium chiglitazar, an innovative compound with independent intellectual property right in China, is intended for the treatment of type II diabetes. It has been confirmed through *ex vivo* and *in vivo* test models that sodium chiglitazar has pharmacological effects of increasing insulin sensitivity, reducing blood glucose and improving other metabolic syndromes. The structural formulas of the two are as follows:

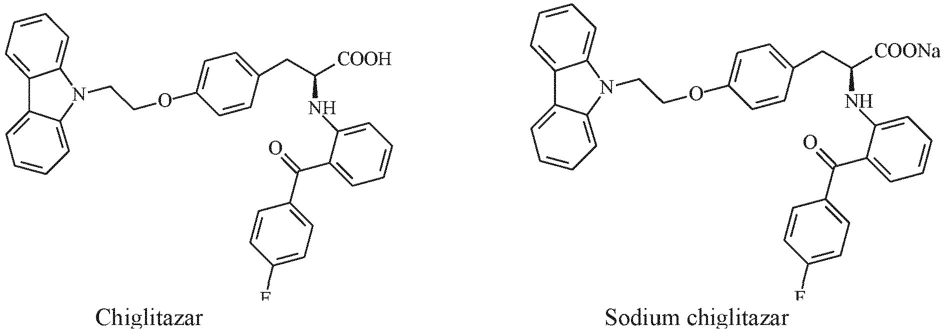

The currently reported indication of chiglitazar is type II diabetes, but there is no relevant report in fibrotic diseases.

Fibrosis is a pathophysiological process that can occur in a variety of tissues and organs in the body. It presents pathological characteristics of the increase of fibrous connective tissue and the decrease of parenchymal cells in tissues and organs. The continuous progression of fibrosis can lead to destruction of organ structure, hypofunction and even failure in organs, which belongs to the pathological type with fatal threat. Organs of the body are composed of parenchyma and interstitium. The parenchymal component refers to the main structure and functional cells of an organ, while the interstitial component is composed of interstitial cells and extracellular matrix, which mainly maintains the morphological structure and normal function of the tissues and organs.

Tissue cell damage caused by exogenous or endogenous factors can induce local inflammatory response and repair process. In the case of minor damage, the repair process includes limited proliferation of functional cells and the supplement of interstitium, while in the case of major damage or the persistence of damage factors, the repair process will also be induced, and the extracellular matrix produced by the activation of interstitial cells would occupy the positions of the parenchymal functional cells, to thereby change the tissue structure and reduce normal physiological functions. Therefore, fibrosis is essentially a normal repair response to the tissue damag to protect the relative integrity of tissues and organs. However, excessive fibrosis can alter the normal morphology of the tissues and organs and weaken their functions.

Almost all major organs of the human body may develop fibrotic lesions, including lung (such as idiopathic interstitial pneumonia, etc.), heart (such as hypertrophic cardiomyopathy, etc.), liver (such as non-alcoholic fatty liver and cirrhosis, etc.), kidney (such as chronic nephritis, etc.), bone marrow (such as myelofibrosis, etc.), skin (such as systemic sclerosis, etc.) and other tissues and organs can be involved.

Fibrotic lesions are a multi-factor and multi-link pathological process, including at least tissue parenchymal cell damage, inflammatory response, interstitial cell activation and matrix formation. Parenchymal cell damage is caused by exogenous factors such as drug damage, and by endogenous factors such as autoimmune damage and metabolic toxicity (e.g. lipotoxicity). The body's normal inflammatory response is usually under control and can be terminated by negative feedback factors after elimination of immune activation factors (e.g. exogenous infection and tissue cell damage). However, chronic inflammation can develop in case of the persistence of stimulating factors (e.g. chronic infection and autoantigens, etc.) or immunodeficiency. Damaged cells and immune cells produce various factors (e.g. transforming growth factor TGFβ or platelet-derived growth factor PDGFα/β, etc.) to stimulate activation and proliferation of interstitial cells, and production of extracellular matrix such as collagen. The persistence of the above stimulation causes matrix accumulation and tissue fibrosis. Therefore, the intervention process of fibrotic diseases requires the participation of multiple links. In current clinical treatment, fibrosis is still an irreversible disease progression. Conventional treatments include corticosteroids and other anti-inflammatory drugs, chemotherapeutic drugs, and anti-platelet-derived growth factor receptor drugs, which usually only partially improve the symptoms of patients. The main goal of such treatments for fibrosis is still to delay the disease progression and maintain normal physiological functions, and for patients in advanced stages, organ transplantation is the only option for rescue. Therefore, there is a huge clinical demand for clinical treatment.

Document WO 2015/189401 discloses the use of a pan-PPAR agonist in the treatment of fibrotic diseases.

### SUMMARY

In view of this, the purpose of the present disclosure is to provide a series of applications of chiglitazar and related compounds thereof in the field of fibrotic diseases.

In the present disclosure, chiglitazar and related compounds thereof include chiglitazar or its stereoisomers, geometric isomers, tautomers, solvates, crystalline forms, amorphous forms, or pharmaceutically acceptable salts thereof.

The pharmaceutically acceptable salt thereof is sodium chiglitazar or potassium chiglitazar, and their structural formulas are as follows:

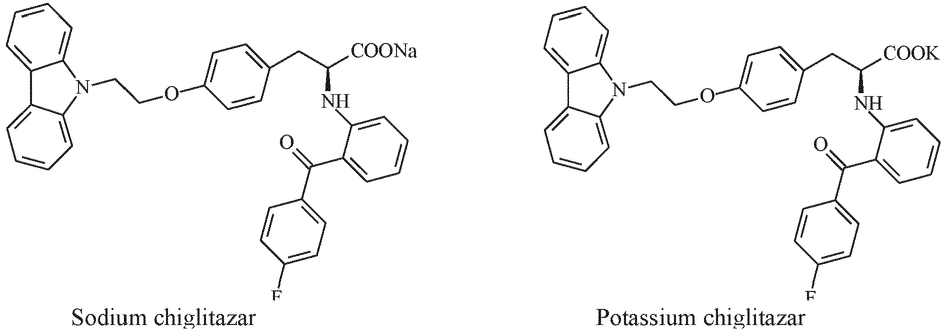

The activation and proliferation of fibroblasts is an important pathological process in the progression of fibrotic diseases. Inhibition of fibroblasts proliferation has a potential to treat fibrotic diseases. The present disclosure used primary human hepatic stellate cells and primary human dermal fibroblasts as test objects to detect the in vitro inhibitory effects of sodium chiglitazar and the reference compounds on both cells. The results showed that sodium chiglitazar had *in vitro* inhibitory activity on the proliferation of human skin- and liver-derived fibroblasts compared with the reference compound, pioglitazone, which showed no obvious inhibitory activity. Chiglitazar sodium specifically inhibits the proliferation of fibroblasts *in vitro*, which is significantly distinct from other known PPAR agonists such as pioglitazone. Based on this, the present disclosure proposes the use of chiglitazar and related compounds thereof in the manufacture of fibroblast inhibitors.

Transforming growth factor β (TGF-β) can activate fibroblasts and induce the expression of extracellular matrix-related genes, which plays an important role in the pathological process of fibrotic diseases. TGF-β1 stimulates fibroblast activation and induces expression of matrix, including α-smooth muscle actin (α-SMA) and connective tissue growth factor (CTGF), by interaction with TGF-β receptors. In the present disclosure, primary human hepatic stellate cells were cultured in a culture medium containing TGF-β1 and chiglitazar sodium or a reference compound, and then the gene expression levels of α-smooth muscle actin (α-SMA) and connective tissue growth factor (CTGF) were detected. The results show that with the stimulation of transforming growth factor TGFβ, the expression of matrix-related genes in fibroblasts was significantly increased, which was significantly inhibited by sodium chiglitazar. For the expression of α-smooth muscle actin, the PPARα/δ dual agonist GFT505 had a certain inhibitory effect, but it is still not as good as sodium chiglitazar, while the PPARγ/α dual agonist pioglitazone had no effect. For the expression of connective tissue growth factor, only sodium chiglitazar had a significant and dose-dependent inhibitory effect. It demonstrated that sodium chiglitazar had a better and unexpected inhibitory effect on fibroblast proliferation and fibrosis-related gene expression than other PPAR agonists such as pioglitazone and GFT505. Based on this, the present disclosure proposes the use of chiglitazar and related compounds thereof in the manufacture of an inhibitor of TGFβ-mediated activation of fibroblast extracellular matrix.

Monocytes are involved in the local inflammation induced by cell injury. They are induced by chemokines released by the injury to reach the injured site, and are activated into macrophages, which in turn produce various inflammatory factors (such as tumor necrosis factor TNFα and monocyte chemoattractant protein MCP-1, etc.) to initiate subsequent inflammatory response, and the persistent inflammatory response induces fibroblast activation and production of extracellular matrix, which is an important pathological process of fibrotic diseases. Inhibition of this process has therapeutic uses for fibrotic diseases. The present disclosure took human acute monocytic leukemia mononuclear cell line (THP-1) as the test object, which were activated by a certain concentration of phorbol esters (Phorbol-12-myristate-13-acetate, PMA), and differentiated into macrophages. Through this cell model, the activating effects of chiglitazar sodium and other compounds on the expression of THP-1 related inflammatory factors were detected. The results show that both chiglitazar sodium and the control compound GFT505 can significantly inhibit phorbol esters-mediated activation of monocyte and the related gene expression, but the effect of the control compound GFT505 was less than that of chiglitazar sodium. Based on this, the present disclosure proposes the use of chiglitazar and related compounds thereof in the manufacture of an inflammatory factor inhibitor.

Monocyte chemotaxis in the blood circulation induced by chemokines to the injured site, thereby to initiate and participate in the subsequent inflammatory response, is one of the important pathological processes of tissue fibrosis. The present disclosure simulated this process by using Transwell technology, and tested the effect of sodium chiglitazar or control compounds on the chemokines-induced chemotaxis of monocytic cell line THP-1. The results showed that sodium chiglitazar significantly inhibited the migration activity (transwell) of monocyte THP-1 induced by monocyte chemokines compared with the control compounds. Pioglitazone had partial inhibitory activity, while the other two control compounds showed no obvious inhibitory activity. Different PPAR agonists show different activity characteristics, and sodium chiglitazar has a stronger inhibitory activity. Based on this, the present disclosure proposes the use of chiglitazar and related compounds thereof in the manufacture of a monocyte chemotaxis inhibitor.

Endogenous or exogenous injury factors (such as chemical toxicants) cause tissue cell death and induce inflammatory response and tissue repair, which is a typical pathology of fibrotic diseases. Carbon tetrachloride (CCl₄) is usually used as a chemical injury factor to simulate liver fibrosis, and also widely used in research model of rodent liver fibrosis. The present disclosure used this model to test the inhibitory effect of sodium chiglitazar at different doses on the liver fibrosis. The results show that in the mouse model, carbon tetrachloride (CCl₄) can induce liver parenchymal cell death, inflammatory infiltration and tissue fibrosis, which is consistent with clinical pathological phenomenon. Sodium chiglitazar at high, medium and low doses can all partially inhibit the inflammatory activity and fibrosis degree of liver tissue, and thus has pharmacodynamic activity for the treatment of fibrotic diseases. Based on this and the aforementioned experimental results, the present invention proposes chiglitazar or its stereoisomers, geometric isomers, tautomers, solvates, crystal forms, amorphous forms, or pharmaceutically acceptable salts for use for treating fibrotic diseases caused by tissue cell damage resulting from chronic inflammation.

Based on a series of experimental results of the present disclosure, it can be concluded that chiglitazar and related compounds thereof have clear effects in the treatment of fibrotic diseases, especially the fibrotic disease caused by tissue cell damage resulting from chronic inflammation. The fibrotic diseases caused by tissue cell damage resulting from chronic inflammation include chronic nephritis and renal fibrosis. myelofibrosis, and non-alcoholic fatty liver

It can be known from the above technical solutions that through the in vitro models and animal models, the present disclosure illustrated that chiglitazar and related compounds thereof inhibited fibroblast activation, matrix production, monocyte activation and chemotactic activity *in vitro,* and reduced inflammatory activity and tissue fibrosis area in animal models of liver fibrosis. Compared with known similar compounds, sodium chiglitazar showed more significant and different activity characteristics, and had a better application potential for treating fibrotic diseases.

### BRIEF DESCRIPTION OF DRAWINGS

FIG. 1 shows the *in vitro* growth inhibition curve of human hepatic stellate cells mediated by chiglitazar sodium (EdU assay);
FIG. 2 shows the in vitro growth inhibition curve of human hepatic stellate cells mediated by chiglitazar sodium (MTT assay) ;
FIG. 3 shows the in vitro growth inhibition curve of human dermal fibroblasts mediated by chiglitazar sodium (MTT assay);
FIG. 4 shows the effects of chiglitazar sodium at various concentrations and the control compounds GFT505 and pioglitazone on the expression of target genes in TGFβ-activated fibroblasts;
FIG. 5 shows the effects of sodium chiglitazar and the control compound GFT505 on the expression of target genes in phorbol esters-activated monocytes, where the bar in each group from left to right is the solvent control (dimethyl sulfoxide), sodium chiglitazar (3 µmol/L) and GFT505 (3 µmol/L) , respectively;
FIG. 6 shows the effects of sodium chiglitazar and the control compounds on the chemotaxis and migration of THP-1 cells (shown as crystal violet staining cells), where A is the solvent control (dimethyl sulfoxide), B is the solvent control + MCP-1 (10 ng/mL), C is sodium chiglitazar (3 µmol/L) + MCP-1 (10 ng/mL), D is GFT505 (3 µmol/L) + MCP-1 (10 ng/mL), E is pioglitazone (3 µmol/L) + MCP-1 (10 ng/mL), and F is rosiglitazone (3 µmol/L) + MCP-1 (10 ng/mL), respectively;
FIG. 7 shows the microscopic images of liver tissue pathological sections stained with H&E;
FIG. 8 shows the inflammation-based scores of liver tissue pathological sections stained with H&E;
FIG. 9 shows the microscopic images of liver tissue pathological sections stained with Picrosirius Red; and
FIG. 10 shows the fibrosis scores of liver tissue pathological sections stained with Picrosirius Red.

### DETAILED DESCRIPTION

The present disclosure discloses the application of chiglitazar and related compounds thereof. Those skilled in the art can learn from the contents of the present disclosure and appropriately improve the processing parameters. It should be particularly indicated that, all similar replacements and changes are obvious for those skilled in the art, which are deemed to be included in the present disclosure.

The following provides a further description of the application of chiglitazar and related compounds thereof provided by the present disclosure.

### Example 1: Activation of human PPAR receptor by sodium chiglitazar

The PPAR reporter gene model comprises three subtypes of expression plasmids, RXR expression plasmid, plasmid with luciferase reporter gene, and GFP expression plasmid as an external reference. The human hepatocyte cell line L-02 cells were seeded into 96-well plates at the density of 15,000 cells/well with about 50% plating rate, followed by culturing for 24 hours at 37°C. The cells in each well were transfected with the corresponding expression plasmid (pHD luciferase reporter gene plasmid + PPARα expression plasmid + GFP expression plasmid for PPARα activity detection; pACOX luciferase reporter gene plasmid + PPARγ expression plasmid + RXR expression plasmid + GFP expression plasmid for PPARγ activity detection; and pGL3-PPRE luciferase reporter gene plasmid + PPARδ expression plasmid + RXR expression plasmid + GFP expression plasmid for PPARδ activity detection) by using FuGENE 6 of Roche.

After transfection for 48 hours, various concentrations of the drugs to be tested were added to the cultured cells with DMSO as the solvent control, and three replicate wells were set for each treatment group.

After adding drugs for 24 hours, the cell culture media were discarded, and removed completely by putting the 96-well plates upside down on absorbent paper, and then 80 µL of cell lysates (Promega, E153A) were added to each well. The cells were allowed to stand for 5 minutes at room temperature before using a pipette to mix well. 60 µL of cell lysates from each well was transferred to the white plates for detection (Corning, 3693).

The fluorescence intensity (at wavelength 485-527 nm) of green fluorescent protein (GFP) in each well was firstly detected as an internal standard by a fluorescence detector, and then 30 µL of luciferase substrates (Promega, E151A) were added to each well. After slightly shaking to mix well, the absorbance values at 562 nm wavelength were detected. The relative reporter gene activation intensity was obtained by the ratio of the fluorescence detection signal of the drugs to be tested to that of the solvent control, with both the signals normalized to the internal standard GFP signal. The half-maximal activation (AC₅₀) of the drugs to be tested was calculated with the activity at various concentrations (shown in Table 1).

**Table 1. Activation activity of sodium chiglitazar on three subtypes of PPAR receptors in the reporter gene model.**

| Half-maximal activation concentration of reporter gene AC₅₀ (µM) | PPARα | PPARγ | PPARδ |
|---|---|---|---|
| chiglitazar sodium | 1.096 | 0.091 | 0.708 |

The result showed that sodium chiglitazar had activation activity on all three subtypes of PPAR.

### Example 2: Inhibition of fibroblasts proliferation by sodium chiglitazar

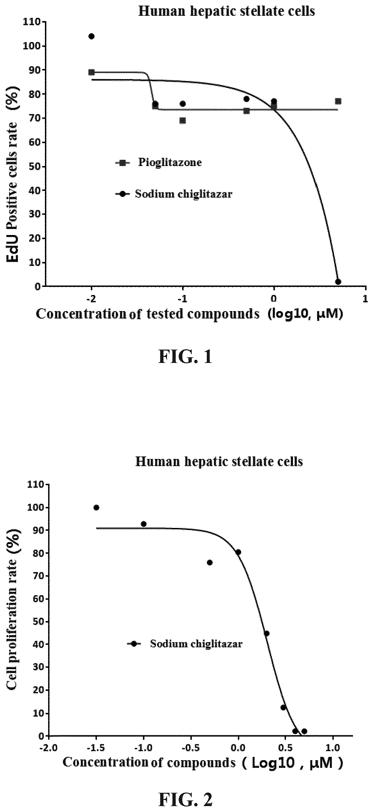

Primary human hepatic stellate cells (HSC) purchased from Sciencell Research Laboratories, Inc. were seeded into 96-well plates with complete growth media (Stellate Cell Medium) for 24 hours, followed by culturing overnight in culture media free of serum and growth factors, and then the culture media were replaced by fresh media containing platelet-derived growth factor (PDGF-BB) and the compounds to be tested (chiglitazar sodium or the reference compound PPARγ/α dual agonist pioglitazone purchased from Selleck Chemicals LLC) at various concentrations, and the cells were cultured for 24 hours. When the cells were treated with the compounds for the last 17 hours, EdU (5-ethynyl-2'-deoxyuridine) was added to the cells. Finally, the culture media were removed and the cells were fixed with formaldehyde. The amount of the EdU incorporated into the DNA of the cells was quantified by fluorescent Click-it EdU assay according to the manufacturer's instructions (Beyotime). The results were showed as the percentage of EdU-positive cells in the total number of cells by using the mean value of three biological replicates. The inhibition of cell proliferation by the compounds was determined by comparing the EdU incorporated value of the control group (FIG. 1).

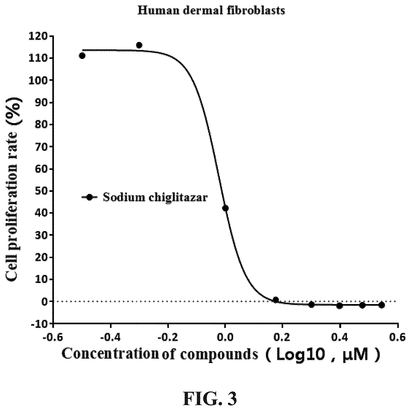

Primary human hepatic stellate cells or primary human dermal fibroblasts (HDF), both purchased from Sciencell, were seeded into 96-well plates with complete growth media (Stellate Cell Medium or Fibroblast Cell Medium) for 24 hours, followed by culturing overnight in the culture media free of serum and growth factors, and then the culture media were replaced by fresh medium containing platelet-derived growth factor (PDGF-BB) and the compounds to be tested at various concentrations, and the cells was cultured for another 72 hours. Measurement was performed using a conventional MTT assay kit (Promega). The results were showed as the relative absorbance (OD490) to the MTT substrate by using the mean value of three biological replicates. The inhibition of cell proliferation by the compounds was determined by comparing the absorbance value of the control group (FIGs. 2, 3).

It can be seen from FIGs. 1-3 that sodium chiglitazar had inhibitory activity on the in vitro proliferation of human skin- and liver-derived fibroblasts compared with the reference compound pioglitazone, which showed no obvious inhibitory activity. Chiglitazar sodium specifically inhibits the *in vitro* proliferation of fibroblasts, which is significantly distinct from other known PPAR agonists such as pioglitazone.

### Example 3: Effect of sodium chiglitazar and the control compounds on expression of transforming growth factor TGFβ-mediated activation of fibroblast genes

Human primary hepatic stellate cells were seeded into 6-well plates with complete growth media (Stellate Cell Medium) for 24 hours, and then the culture media were replaced by fresh media (free of serum and growth factors) containing TGF-β1 and the compound to be tested (sodium chiglitazar) or the reference compound PPARγ/α dual agonist pioglitazone or PPARα/δ dual agonist GFT505 (both purchased from Selleck), and the cells were cultured for 24 hours. The total RNAs of the samples were extracted, and reverse transcription reaction was performed. Thereafter, a conventional PCR amplification kit (FastStart Universal SYBR@ Green Master (ROX), Roche) was used for further detection, where the PCR primers were designed with reference to the NCBI standard mRNA sequence of the target genes (Table 2). PCR reaction and detection were carried out on the ABI StepOnePlus real-time quantitative PCR instrument.

**Table 2. Target gene primers used for detection (including internal reference gene)**

| Primer name | Primer sequence (5' to 3') |
|---|---|
| α-smooth muscle actin (α-SMA) forward primer | CGTGGGTGACGAAGCACAG |
| α-smooth muscle actin (α-SMA) reverse primer | GGTGGGATGCTCTTCAGGG |
| Connective tissue growth factor (CTGF) forward primer | GGAGTGGGTGTGTGACGAG |
| Connective tissue growth factor (CTGF) reverse primer | GACCAGGCAGTTGGCTCTAA |
| β-actin (ACTB) forward primer | CGGGAAATCGTGCGTGAC |
| β-actin (ACTB) reverse primer | GGAAGGAAGGCTGGAAGAG |
| Monocyte chemoattractant protein 1 (MCP-1) forward primer | GATCTCAGTGCAGAGGCTCG |
| Monocyte chemoattractant protein 1 (MCP-1) reverse primer | TTTGCTTGTCCAGGTGGTCC |
| Tumor necrosis factor α (TNFα) forward primer | TTCTCGAACCCCGAGTGACA |
| Tumor necrosis factor α (TNFα) reverse primer | TCTCTCAGCTCCACGCCATT |

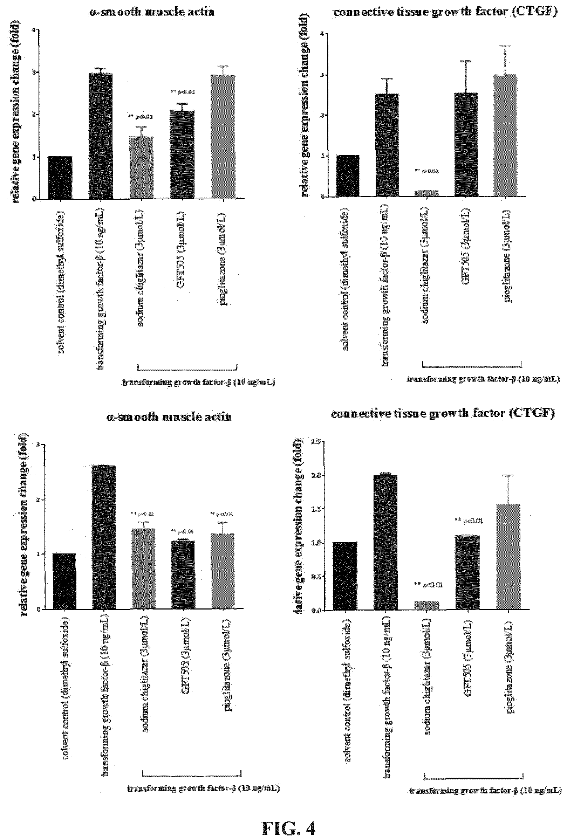

By calculating the relative amount of transcripts was calculate by "Delta-Delta CT method" (Livak et al. Methods 2001) , and using ACTB as the housekeeping gene for normalization and the average data of untreated human hepatic stellate cells as a reference control, the relative change folds of gene expression were obtained (FIG. 4).

The results showed in FIG. 4 indicated that with the stimulation of transforming growth factor TGFβ, the expression of matrix-related genes in fibroblasts was significantly increased, which was significantly inhibited by sodium chiglitazar. For the expression of α-smooth muscle actin, the PPARα/δ dual agonist GFT505 had a certain inhibitory effect, while the PPARγ/α dual agonist pioglitazone had no effect. For the expression of connective tissue growth factor, only sodium chiglitazar had a significant and dose-dependent inhibitory effect. It demonstrated that sodium chiglitazar had a better and unexpected inhibitory effect on fibroblast proliferation and fibrosis-related gene expression than other PPAR agonists such as pioglitazone and GFT505.

### Example 4: Effect of sodium chiglitazar and the control compounds on the expression of related inflammatory factors in phorbol esters-mediated activation of monocytic cell line THP-1

Human acute monocytic leukemia mononuclear cell line (THP-1) cells were activated by a certain concentration of phorbol esters (Phorbol-12-myristate-13-acetate, PMA) and differentiated into macrophages. Through this cell model, the activating effects of sodium chiglitazar and other compounds on the expression of THP-1 related inflammatory factors were detected.

THP-1 cells were seeded into 6-well plates with complete growth media for 24 hours, and then the culture media were replaced by fresh media containing PMA and the compound to be tested (sodium chiglitazar) or the reference compound GFT505 (PPARα/δ dual agonist), and the cells were cultured for 6 hours. The total RNAs of the samples were extracted, and cDNA templates were synthesized by reverse transcription reaction (Transcriptor First Strand cDNA Synthesis Kit, Roche). Thereafter, a conventional PCR amplification kit (FastStart Universal SYBR@ Green Master (ROX), Roche) was used for semi-quantitative gene expression detection on the ABI StepOnePlus real-time quantitative PCR instrument. The target gene primers were shown in Table 2.

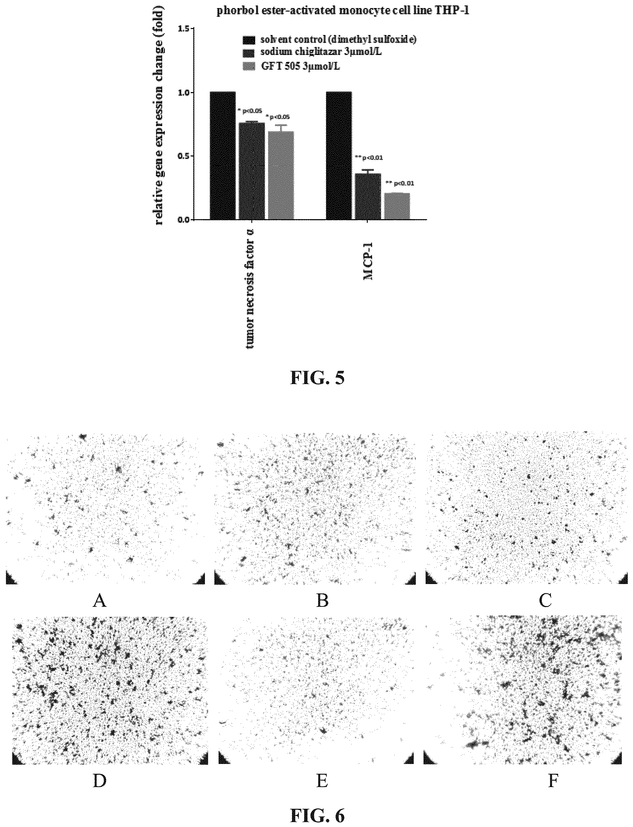

The relative expression change of the target genes was obtained by using the internal reference β-actin gene (ACTB) as the standard for normalization, compared with the untreated cell samples, and the relative expression change folds were calculated (FIG. 5).

The results showed in FIG. 5 indicated that both sodium chiglitazar and the control compound GFT505 can significantly inhibit phorbol esters-mediated activation of monocyte and related gene expression, but the effect of the control compound GFT505 was not as good as sodium chiglitazar.

### Example 5: Effect of chiglitazar sodium and the control compounds on the chemokines-induced chemotaxis of monocytic cell line THP-1

In an in vitro model, migration of the monocytic cell line THP-1 induced by a certain concentration of chemokines (such as monocyte chemotactic protein 1, MCP-1), and accordingly such in vivo process was mimicked . Transwell technology, according to the manufacturer's instructions from Corning Inc., is considered that Traswell is a kind of a polycarbonate membrane filter as well as a permeable holder. The polycarbonate membrane divides a culture chamber into upper and lower ones, where the components of the culture medium in the lower chamber can affect the growth, differentiation and movement of the cells in upper chamber. Finally, the cell migration ability can be reflected by staining and counting the cells in the lower chamber.

When cultured to the log phase stage, THP-1 cells were resuspended, adjusted the concentration, and seeded in 24-well plates, followed by being pretreated with the culture media containing the compound to be tested (sodium chiglitazar) or the reference compound GFT505, pioglitazone, or PPARγ single agonist rosiglitazone (all purchased from Selleck) for 24 hours. Transwells were used according to the manufacturer's instructions. The culture media containing chemokine MCP-1 were added to the lower chamber (i.e., the bottom of the 24-well plates), and the cell suspension was added to the upper chamber and cultured for 4 hours. Later, the cells cultured on the "ceiling" surface of the polycarbonate membrane were fixed and stained with crystal violet solution (FIG. 6), and then the number of cells in the lower chamber was counted (Table 3).

**Table 3 Cell counting in the lower chamber in transwell experiment**

| | | Monocyte chemoattractant protein (MCP-1, 10 ng/mL) | | | | |
|---|---|---|---|---|---|---|
| Compound concentration (µmol/L) | Solvent control (dimethyl sulfoxide, final concentration 0.1%) | | Sodium chiglitazar (3 µmol/L) | GFT505 (3 µmol/L) | Pioglitazone (3 µmol/L) | Rosiglitazone (3 µmol/L) |
| Cell concentration in lower chamber (cells/ml) | 1289 | 24858 | 10630 | 28443 | 17529 | 21804 |
| | 1391 | 27504 | 9760 | 28511 | 17416 | 21883 |
| | 1368 | 26113 | 9646 | 27572 | 17597 | 20752 |
| lean value | 1349 | 26158 | 10012 | 28175 | 17514 | 21480 |
| Migration rate (%) | - | 100 | 33.12 | 102.55 | 61.80 | 76.96 |

It can be seen in Table 3 and FIG. 6 that sodium chiglitazar significantly inhibited the migration activity of monocyte THP-1s induced by the monocyte chemokine compared with the control compounds (transwell). Pioglitazone had partial inhibitory activity, which was obviously not as good as sodium chiglitazar, while the other two control compounds showed no obvious inhibitory activity. Different PPAR agonists show different activity characteristics, and sodium chiglitazar has a stronger inhibitory activity.

### Example 6: Inhibitory effect of chiglitazar sodium in mouse model of carbon tetrachloride induced liver fibrosis

BALB/c mice were injected intraperitoneally with 25% CCl₄ (4 mL/kg body weight) twice a week, which can cause liver damage and chronic liver fibrosis in 3 weeks. The model animals were administered for 6 weeks continuously, and the non-model control group was administered the solvent control olive oil for the same period. Model animals were randomly divided into groups from the third week. The control group was administered solvent (0.2% sodium methylcellulose) intragastrically from the fourth week, and the sodium chiglitazar at high, medium and low dose groups were administered 40, 20, and 10 mg/kg body weight of the active ingredient sodium chiglitazar intragastrically, respectively (Table 4), until the end of the experiment in the 7^{th} week. After the experimental animals were sacrificed, the plasma and liver tissue samples were collected to measure the contents of plasma transaminase, liver tissue weight and the ratio of liver weight to body weight, respectively. The liver tissues were fixed to perform pathological sections, and then hematoxylin-eosin staining (H&E staining) and Picrosirius red staining were conducted, respectively. The former was used to assess cell damage and inflammation, and the latter was used to assess tissue fibrosis.

**Table 4 Test grouping and drug treatment**

| Group | Number of animals |
|---|---|
| G1: Blank control group (olive oil + 0.2% sodium methylcellulose) | 10 |
| G2: Model control (CCl₄+0.2% sodium methylcellulose) | 10 |
| G3: CCl₄+ sodium chiglitazar at low dose (10 mg/kg body weight) | 10 |
| G4: CCl₄+ sodium chiglitazar at medium dose (20 mg/kg body weight) | 10 |
| G5: CCl₄+ sodium chiglitazar at high dose (40 mg/kg body weight) | 10 |

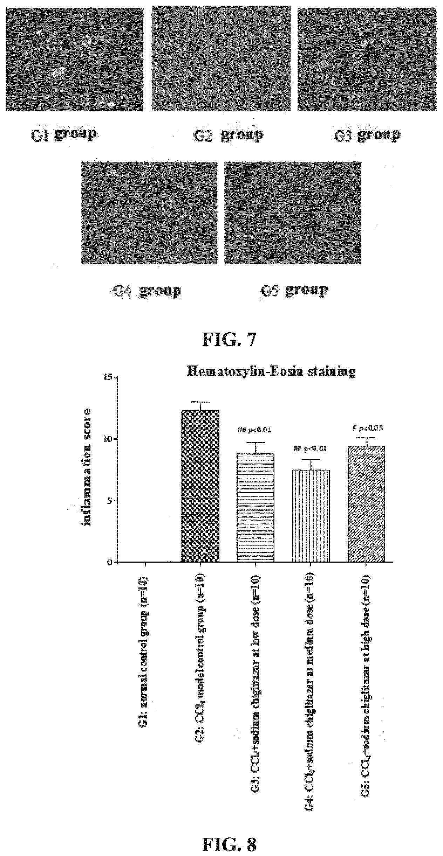

The inflammation-based scores of liver tissues were obtained by microscopic observation and scoring of pathological sections stained with hematoxylin and eosin (H&E staining). Single-blind testing and scoring were conducted by pathologists, using the Metavir scoring system for inflammatory activity, comprising four independent indicators, that is, portal inflammation, lobular inflammation, piecemeal necrosis, and bridging necrosis (FIG. 8).

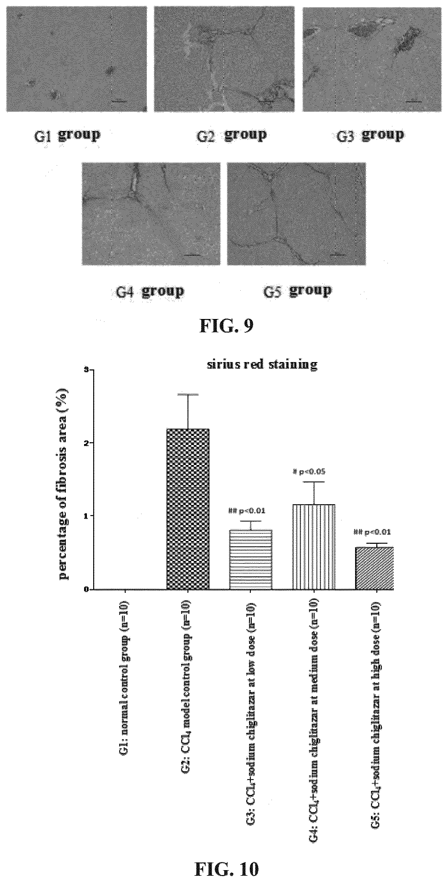

The liver tissue sections were stained with Picrosirius Red for morphological observation (FIG. 9), and measured by using a semi-automatic digital image analysis system and the measurement software (OsteoMetrics, Inc.). The pathologist single-blindly tested and hand-drew the entire sections, and then measured the fibrosis area on the entire sections in straight lines. The software automatically calculated the total area of the measurement. Fibrosis area% = Total fibrosis area ÷ Total liver tissue section area*100 (FIG. 10).

It can be seen from the results in FIGs. 7-10 that in the mouse model, carbon tetrachloride (CCl₄) can induce liver parenchymal cell death, inflammatory infiltration and tissue fibrosis. Sodium chiglitazar at high, medium and low doses can all partially inhibit the inflammatory activity and fibrosis degree of liver tissues, and thus had pharmacodynamic activity of treating fibrotic diseases.

## Claims

1. Chiglitazar or its stereoisomers, geometric isomers, tautomers, solvates, crystal forms, amorphous forms, or pharmaceutically acceptable salts for use in the treatment of a fibrotic disease caused by tissue cell damage resulting from chronic inflammation.

2. Chiglitazar or its stereoisomers, geometric isomers, tautomers, solvates, crystal forms, amorphous forms, or pharmaceutically acceptable salts for use according to claim 1, wherein the pharmaceutically acceptable salt of chiglitazar is sodium chiglitazar or potassium chiglitazar.

3. Chiglitazar or its stereoisomers, geometric isomers, tautomers, solvates, crystal forms, amorphous forms, or pharmaceutically acceptable salts for use according to claim 1, wherein the fibrotic disease caused by tissue cell damage resulting from chronic inflammation includes chronic nephritis and renal fibrosis, myelofibrosis, and non-alcoholic fatty liver.

4. Chiglitazar or its stereoisomers, geometric isomers, tautomers, solvates, crystal forms, amorphous forms, or pharmaceutically acceptable salts for use according to claim 1, wherein the fibrotic disease caused by tissue cell damage resulting from chronic inflammation is hepatic fibrosis.

## Patentansprüche

1. Chiglitazar oder seine Stereoisomere, geometrischen Isomere, Tautomere, Solvate, Kristallformen, amorphen Formen oder pharmazeutisch annehmbaren Salze zur Verwendung bei der Behandlung einer fibrotischen Erkrankung, die durch eine aus einer chronischen Entzündung resultierende Gewebeschädigung verursacht wird.

2. Chiglitazar oder seine Stereoisomere, geometrischen Isomere, Tautomere, Solvate, Kristallformen, amorphen Formen oder pharmazeutisch annehmbaren Salze zur Verwendung nach Anspruch 1, wobei das pharmazeutisch annehmbare Salz von Chiglitazar Natriumchiglitazar oder Kaliumchiglitazar ist.

3. Chiglitazar oder seine Stereoisomere, geometrischen Isomere, Tautomere, Solvate, Kristallformen, amorphen Formen oder pharmazeutisch annehmbaren Salze zur Verwendung nach Anspruch 1, wobei die fibrotische Erkrankung, die durch eine aus einer chronischen Entzündung resultierende Gewebeschädigung verursacht wird, chronische Nephritis und Nierenfibrose, Myelofibrose und nichtalkoholische Fettleber beinhaltet.

4. Chiglitazar oder seine Stereoisomere, geometrischen Isomere, Tautomere, Solvate, Kristallformen, amorphen Formen oder pharmazeutisch annehmbaren Salze zur Verwendung nach Anspruch 1, wobei die fibrotische Erkrankung, die durch eine aus einer chronischen Entzündung resultierende Gewebeschädigung verursacht wird, Leberfibrose ist.

## Revendications

1. Chiglitazar ou ses stéréoisomères, isomères géométriques, tautomères, solvates, formes cristallines, formes amorphes ou sels pharmaceutiquement acceptables pour le traitement d'une maladie fibrotique causée par des lésions cellulaires tissulaires résultant d'une inflammation chronique.

2. Chiglitazar ou ses stéréoisomères, isomères géométriques, tautomères, solvates, formes cristallines, formes amorphes ou sels pharmaceutiquement acceptables pour une utilisation selon la revendication 1, dans lequel le sel pharmaceutiquement acceptable de chiglitazar est le chiglitazar sodique ou le chiglitazar potassique.

3. Chiglitazar ou ses stéréoisomères, isomères géométriques, tautomères, solvates, formes cristallines, formes amorphes ou sels pharmaceutiquement acceptables pour une utilisation selon la revendication 1, dans lequel la maladie fibrotique causée par des lésions cellulaires tissulaires résultant d'une inflammation chronique comprend la néphrite chronique et la fibrose rénale, la myélofibrose et la stéatose hépatique non alcoolique.

4. Chiglitazar ou ses stéréoisomères, isomères géométriques, tautomères, solvates, formes cristallines, formes amorphes ou sels pharmaceutiquement acceptables pour une utilisation selon la revendication 1, dans lequel la maladie fibrotique causée par des lésions cellulaires tissulaires résultant d'une inflammation chronique est une fibrose hépatique.
